# EUROPÄISCHE PATENTSCHRIFT

(11) **EP 1 030 642 B1**
(45) Veröffentlichungstag und Bekanntmachung des Hinweises auf die Patenterteilung: **12.03.2003**
(21) Anmeldenummer: 98954469.7
(22) Anmeldetag: 30.10.1998
(51) Int. Cl.: A61K 7/00, A61K 7/48, A61K 7/32

(54) **MEHRPHASEN-STIFTPRÄPARAT**
MULTIPHASE STICK PREPARATION
PREPARATION MULTIPHASE SOUS FORME DE STICK

(30) Priorität: 11.11.1997 DE 19749760
(43) Veröffentlichungstag der Anmeldung: 30.08.2000
(73) Patentinhaber: Henkel Kommanditgesellschaft auf Aktien, 40589 Düsseldorf (DE)
(72) Erfinder: BANOWSKI, Bernhard, D-40597 Düsseldorf (DE); SCHOLZ, Wolfhard, D-47829 Krefeld (DE); BORDAT, Pascal, F-31130 Flourens (FR); ZINKEN, Marion, D-41564 Kaarst (DE)
(86) Internationale Anmeldenummer: EP9806892
(87) Internationale Veröffentlichungsnummer: WO99023998

(56) Entgegenhaltungen:
- EP-A- 0 701 812
- DE-B- 1 122 221
- US-A- 4 120 948

## Beschreibung

Die Erfindung betrifft eine kosmetische oder dermatologische Zubereitung in Form eines Stiftes zur Abgabe einer bei Umgebungstemperatur formstabilen, bei Körpertemperatur verstreichbaren Masse an die Haut, die zwei oder mehrere unterschiedlich zusammengesetzte Gelphasen aufweist.

Zweiphasen-Stiftzubereitungen auf Basis alkoholischer Seifengele sind seit langem, z.B. aus DE-AS 1 122 221 bekannt. Solche Zubereitungen bieten die Möglichkeit, einzelne Bestandteile, die mit anderen Komponenten unverträglich sind, in eine Phase des Stiftes einzubringen und auf diese Weise unerwünschte Wechselwirkungen mit den Komponenten der zweiten Phase zu verhindern. Auch kann man die Lagerbeständigkeit der Stiftpräparate dadurch erhöhen, daß man leichter flüchtige oder oxidationsempfindliche Komponenten in den Kern oder line der inneren Phasen des Stiftes einbringt.

Ein Problem besteht aber darin, daß der Kontakt zwischen den beiden Phasen zu Wechselwirkungen zwischen den Phasen, insbesondere zu einem Ausbluten einzelner Komponenten in die andere Phase führen kann. Um dieses zu verhindern, wurde in DE-A-2 752 420 ein Zweiphasen-Antitranspirant-Stift vorgeschlagen, der einen Kern aus einem mit Wachsen verfestigten Öl und eine Hülle aus einen Polyol-Seifengel aufweist.

Diese bekannten Stiftpräparate sind sowohl sensorisch als auch anwendungstechnisch nicht befriedigend. Sie lösen vor allem nicht das Problem, bei Zweiphasen-Seifengel-Stiften ein Ausbluten einzelner Komponenten aus einer Phase in die andere wirksam zu verhindern.

Darüber hinaus bestand die Aufgabe, Mehrphasen-Stiftpräparate mit einem besonders attraktiven, ästhetisch befriedigenden Aussehen und verbesserten sensorischen Eigenschaften zu entwickeln.

Diese Aufgabe wurde erfindungsgemäß dadurch gelöst, daß in einer der Gelphasen ein poröses Pulver aus sphärischen Polymerteilchen dispergiert wurde. Solche Mikrosphären bieten auch die Möglichkeit, diese mit empfindlichen Wirkstoffen, leichtflüchtigen Duftstoffen oder mit Farbstoffen zu beladen und auf diese Weise eine Stabilisierung solcher Komponenten zu erreichen.

Gegenstand der Erfindung ist daher ein Stiftpräparat aus einer bis 40° C formstabilen, auf der Haut verstreichbaren und bei Temperaturen oberhalb 40° C schmelzbaren Masse, die aus zwei oder mehreren getrennten, unterschiedlich zusammengesetzten Gelphasen besteht, die ein- oder mehrwertige Alkohole, Gelierungsmittel, Duftstoffe, kosmetische oder dermatologische Wirkstoffe sowie gegebenenfalls Wasser und galenische Hilfsmittel enthalten, wobei in einer der Gelphasen 0,1 bis 10 Gew.-%, bezogen auf diese Phase, eines porösen Pulvers aus sphärischen Polymerteilchen dispergiert ist.

Das erfindungsgemäße Stiftpräparat kann man darüber hinaus durch unterschiedliche Transparenz, Färbung oder Pigmentierung der Phasen ästhetisch sehr attraktiv gestalten. Die sphärischen Polymerteilchen verbessern das Haugefühl bei der Anwendung solcher Stifte auf der Haut durch eine Erhöhung der Gleitwirkung und eine Verminderung der Klebrigkeit. Schließlich wurde beobachtet, daß Duftstoffe und kosmetische Wirkstoffe zumindest teilweise in den porösen Polymerteilchen absorbiert werden und kontrolliert, also über einen längeren Zeitraum, auf der Haut wieder freigegeben werden. Dieser Retard-Effekt läßt sich noch dadurch verstärken, daß man die porösen Polymerteilchen bei der Herstellung zunächst mit den Duftstoffen und Wirkstoffen belädt und so in die Gelphase einbringt.

Als Gelphase im Sinne der Erfindung sind Zusammensetzungen zu verstehen, die eine flüssige Phase umfassen, die durch ein Gelierungsmittel verfestigt worden ist. Die flüssige Phase kann dabei aus Wasser, einwertigen und mehrwertigen Alkoholen mit 2 bis 8 C-Atomen und Gemischen daraus bestehen. Als Mittel zur Gelierung dieser flüssigen Phasen eignen sich oberflächenaktive Stoffe, die in der flüssigen Phase gelöst eine Netzwerkstruktur ausbilden und so die flüssige Phase zum Gel verfestigen. Solche Gelierungsmittel sind z.B. die Metallsalze von Fettsäuren, bevorzugt mit 12 bis 22 C-Atomen, Fettsäureamide, Fettsäurealkanolamide, Dibenzalsorbit und bestimmte Polymere, z.B. alkohollösliche Polyamide und Polyacrylamide oder Mischungen solcher Gelierungsmittel. Bevorzugte Gelierungsmittel sind die Alkali-, Erdalkali-, Aluminiumund Aminseifen von C₁₂ - C₂₂ - Fettsäuren, z.B. Natriumstearat, Natrium-palmitat, Magnesiumstearat oder Aluminium-stearat.

Als mehrwertige Alkohole eignen sich bevorzugt Polyole mit 2 bis 8 C-Atomen und 2 bis 6 Hydroxylgruppen, z.B. Ethylenglycol, 1,2-Propylenglycol, 1,3-Propylenglycol, 1,2-Butylenglycol, 1,3-Butylenglycol, 1,4-Butylenglycol, 2-Methyl-propandiol-1,3, Glycerin, Erythrit, Pentaerythrit, Trimethylolpropan, Sorbit, Methylglucosid, Cyclohexantriol oder Inosit. Als einwertige Alkohole eignen sich z.B. Ethanol, n-Propanol und Isopropanol. Bevorzugte Komponenten der flüssigen Phase sind Ethanol, 1,2-Propylenglycol, 1,3-Butandiol, Glycerin, Sorbit und Gemische davon, gegebenenfalls auch im Gemisch mit Wasser.

Darüber hinaus enthalten die Gelphasen Duftstoffe oder kosmetische oder dermatologische Wirkstoffe.

Als Duftstoffe bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen zB. die Jonone, ∝-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen oder tierischen Quellen zugängich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als kosmetische Wirkstoffe kommen vor allem solche Substanzen in Frage, die einen günstigen Einfluß auf die ästhetischen Eigenschaften der Haut, insbesondere auf ihre Glätte und Geschmeidigkeit, auf die Hautfeuchtigkeit, auf Schweißabsonderung und Körpergeruch sowie auf ihre Färbung bzw. Bräunung und den Schutz vor den schädigenden Einflüssen der Umwelt, insbesondere des Sonnenlichts, ausüben.

Bevorzugte kosmetische Wirkstoffe für die erfindungsgemäßen Stiftzubereitungen sind vor allem deodorierende und transpirationshemmende Stoffe. Als solche werden vor allem antimikrobielle Stoffe verstanden, die eine hemmende Wirkung auf schweißzersetzende Mikroorganismen ausüben oder enzymhemmende Stoffe, die das schweißzersetzende Esterase-Enzym hemmen. Geeignete antimikrobielle Stoffe sind z.B. 2,4,4'-Trichlor-2'hydroxydiphenylether (Triclosan®), Chlorhexidin-gluconat, Phenoxyethanol, 1,5-Pentandiol, 1,6-Hexandiol, antimikrobielle ätherische Öle oder Farnesol. Geeignete lipasehemmende Wirkstoffe sind z.B. Triethylcitrat oder Triacetin. Auch schweißhemmende adstringierende Stoffe, die mit der Gelphase verträglich sind, können in den Stiftpräparaten enthalten sein. Solche geeigneten Antitranspirant-Wirkstoffe sind z.B. Natrium-Aluminium-chlorhydroxylactat, das unter der Bezeichnung Chloracel® im Handel ist, und andere adstringierende Substanzen.

Als dermatologische Wirkstoffe werden im allgemeinen solche Substanzen verstanden, die einen heilenden oder vorbeugenden Einfluß auf krankhafte Zustände der Haut haben.

Solche geeigneten dermatologischen Wirkstoffe sind z.B. Lokalanästhetika, Antibiotika, Antiphlogistika, Antiallergica, Corticosteoide, Sebostatika oder andere lokal wirksame pharmazeutische Stoffe.

Auch Vitamine, Panthenol, Allantoin, Pflanzenextrakte und Proteine mit dermatoligischer Wirkung, z.B. auch Octoxyglycerin, können als Wirkstoffe enthalten sein.

Poröse Pulver aus sphärischen Polymerteilchen werden seit langem in der Kosmetik als Komponente von Hautpflegemitteln verwendet, da sie die Hautglätte günstig beeinflussen und die Klebrigkeit verhindern können (vgl. EP 105 657 A1 und EP 409 690 B1). Es sind auch verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus dem Teilchen heraus diffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester und Polyamide.

Die Herstellung von porösen Mikrokugeln durch spezielle Polymerisationsverfahren ist z.B. für Polyamide in DE-A-2 160 135, EP 192 515 B1 und EP 303 530 B1 beschrieben. Nach den dort beschriebenen Verfahren kann man auch Füllstoffe, z.B. Pigmentpartikel, dem Polymerisationsansatz zufügen und auf diese Weise mit Füllstoffen oder Pigmentpartikeln gefüllte, poröse Mikrokügelchen aus Polyamiden erzeugen. Ein Verfahren zur Herstellung von Mikrokugeln aus inerten Partikeln, z.B. Pigmenten, die mit Polyamid umhüllt sind, ist auch in EP 196 972 B1 beschrieben.

Bevorzugte poröse Pulver aus sphärischen Polymerteilchen enthalten einen Kern aus einem Pigmentpartikel, z.B. aus Titandioxid. Durch solche Polymerpulver kann man in die klare Gelphase einen weißen oder farbigen gegebenenfalls perlmuttglänzenden Farbeffekt einbringen, der dem erfindungsgemäßen Zweiphasen-Stiftpräparat ein attraktives Aussehen verleiht.

Die porösen Pulver aus sphärischen, gegebenenfalls einen Pigmentkern aufweisenden Polymerteilchen weisen bevorzugt eine mittlere Korngröße von 0,5 - 50 µm und eine spezifische Oberfläche von 1 - 20 m²/g auf. Solche Polymerpulver sind im Handel erhältlich, z.B. aus Polyethylen unter der Bezeichnung Microthene® (U.S.J. Chemicals), aus Polyvinylidenchlorid unter der Bezeichnung Miralite® (Pierce & Stevens Chem. Corp.) und aus Polyamide (Nylon) unter der Bezeichnung Orgasol© (ATOCHEM SA.). Weitere bekannte Handelsprodukte sind z.B. Polyacrylate (Polytrap© von Dow Corning), Polymethacrylate (Micropearl© von SEPPIC), Polyethylene und Polypropylene (Accurel® von Akzo).

Ein erfindungsgemäß besonders bevorzugtes Zweiphasen-Stiftpräparat enthält sphärische Polymerteilchen mit einem Kern aus Pigment, bevorzugt Titandioxid, in einer Menge von 30 bis 60 Gew.-%, bezogen auf Teilchenmasse. Ein solches Produkt auf der Basis von Polyamid ist z.B. als Orgasol® 1002 Ex D Weiß 10 Cos von der Fa. Lehmann & Voss & Co, Hamburg zu beziehen.

Die Gelphasen der erfindungsgemäßen Mehrphasen-Stifte bestehen bevorzugt aus Seifengelen und enthalten

| | |
|---|---|
| 20 - 90 Gew.-% | einwertige oder mehrwertige Alkohole mit 2 - 6 C-Atomen |
| 4 - 14 Gew.-% | Fettsäuren mit 12 - 22 C-Atomen in Form ihrer Metall- oder Aminseifen |
| 0,1 - 30 Gew.-% | Duftstoffe oder kosmetische oder dermatologische Wirkstoffe |

sowie gegebenenfalls Wasser und übliche galenische Hilfsmittel.

Als übliche galenische Hilfsmittel sind solche Stoffe anzusehen, die in Stiftpräparaten dieser Art üblicherweise zur Beeinflussung von Konsistenz, Transparenz, Abrieb oder Stabilität zugegeben werden. Solche Hilfsmittel sind vor allem oberflächenaktive Stoffe, z.B. Emulgatoren, Solubilisatoren und Dispergatoren. Weiterhin werden Verdickungsmittel, z.B. wasserlösliche Polymere, Schichtsilikate, pyrogene Kieselsäure, Elektrolytsalze wie KCl, NaCl, Komplexbildner, z.B. EDTA-Tetranatriumsalz, und andere Hilfsmittel verwendet.

Bevorzugt sind die Gelphasen des erfindungsgemäßen Mehrphasen-Stiftpräparats sehr ähnlich zusammengesetzt und unterscheiden sich im wesentlichen nur durch Farbe, Transparenz, den Gehalt an Polymerpulver und die darin enthaltenen Wirkstoffe.

Es ist weiterhin bevorzugt, das poröse Pulver aus sphärischen Polymerteilchen mit Duftstoffen oder kosmetischen bzw. dermatologischen Wirkstoffen zu beladen. Dies geschieht bevorzugt in der Weise, daß die Mikrosphären in Form einer Dispersion in einoder mehrwertigen Alkoholen, in der auch die Duftstoffe oder Wirkstoffe enthalten sind, in die Gelmasse eingearbeitet werden.

Auf diese Weise wird eine verzögerte Freisetzung der Duft- und Wirkstoffe auf der Haut - und damit eine Verlängerung der Wirkung auf der Haut erreicht. Auch wird dadurch eine unerwünschte Wechselwirkung mit anderen Komponenten der Gelphase verhindert.

Man kann auf diese Weise auch ganz unterschiedliche oder miteinander nicht verträgliche Wirkstoffe in die Stiftmasse einarbeiten, wenn man einen Teil des Polymerpulvers mit dem einen Wirkstoff und einen anderen Teil des Polymerpulvers mit einem anderen Wirkstoff belädt und die so unterschiedlich beladenen Polymerpulver in die gleiche Phase der Stiftmasse einbringt.

In einer besondes-bevorzugter. Ausführung liegt die Masse in Form von zwei oder mehr konzentrisch angeordneten Phasen vor, von welchen die innere oder eine der inneren Phasen das dispergierte Pulver aus sphärischen Polymerteilchen enthält. Die äußere Phase ist bevorzugt transparent und gegebenenfalls schwach gefärbt. Auf diese Weise werden besonders ästhetisch ansprechende Stiftpräparate erhalten. Solche Stiftpräparate kann man z.B. in der Weise produzieren, daß man zunächst den Kern durch Gießen des in der Wärme verflüssigten Gels in eine Form, Erkalten lassen unter Gelierung, Entformen, Einbringen in eine breitere Form und dann die Hülle durch Eingießen des in der Wärme verflüssigten Gels in den Zwischenraum zwischen Kern und Formwand, Erkalten lassen unter Gelierung und Entformen, herstellt.

Ein analoges Verfahren besteht darin, daß man zunächst die Hülle durch Eingießen des verflüssigten Gels in eine Ringform mit entfernbarem zylindrischen Kernstück, Erkalten lassen unter Gelierung und Entfernen des Kernstücks, herstellt und dann die verflüssigte Gelmasse des Kerns in den zylindrischen Hohlraum der so hergestellten Hülle eingießt und erstarren läßt.

Der Kern muß prinzipiell nicht zylindrisch geformt sein, sondern kann auch die Form eines Konus bzw. Kegelstumpfes oder einer Schnecke aufweisen. Aus Gründen der Fertigungstechnik kann es bevorzugt sein, daß die Phasen parallel zur Längsachse des Stiftes angeordnet sind.

In diesem Falle kann man nämlich prinzipiell die aus der Technologie der Seifenherstellung bekannten kontinuierlichen Verfahren zur Herstellung von mehrfarbigen Seifenstränge aus unterschiedlich gefärbten Seifenmassen einsetzen, um einen Strang aus zwei oder mehr verschiedenen Gelmassen herzustellen, der in Stifte beliebiger Länge geschnitten werden kann. Solche Verfahren zur Herstellung von Strängen aus konzentrisch angeordneten Phasen sind z.B. die aus AT-PS 198 501 oder DE-AS-2 526 917 bekannten Koaxial-Strangpreßverfahren. Andere Verfahren zur Herstellung mehrphasiger Stränge aus parallel zur Längsachse angeordneten (aber nicht konzentrischen) Phasen sind in US 3,268,970 beschrieben.

Die auf diese Weise hergestellten Mehrphasen-Stiftpräparate werden bevorzugt in eine Hülse mit einem entlang der Hülsenachse durch Schub-, Dreh- oder Druckmechanik verschiebbarem Bodenkolben eingebracht, wie sie für Deodorant-Stifte und andere kosmetische Stifte üblich sind. Dadurch wird eine bequeme Anwendung des Stifts ohne direkten Kontakt der Finger mit der Stiftmasse ermöglicht.

Die folgenden Beispiele sollen die Erfindung näher erläutern:

### Beispiele

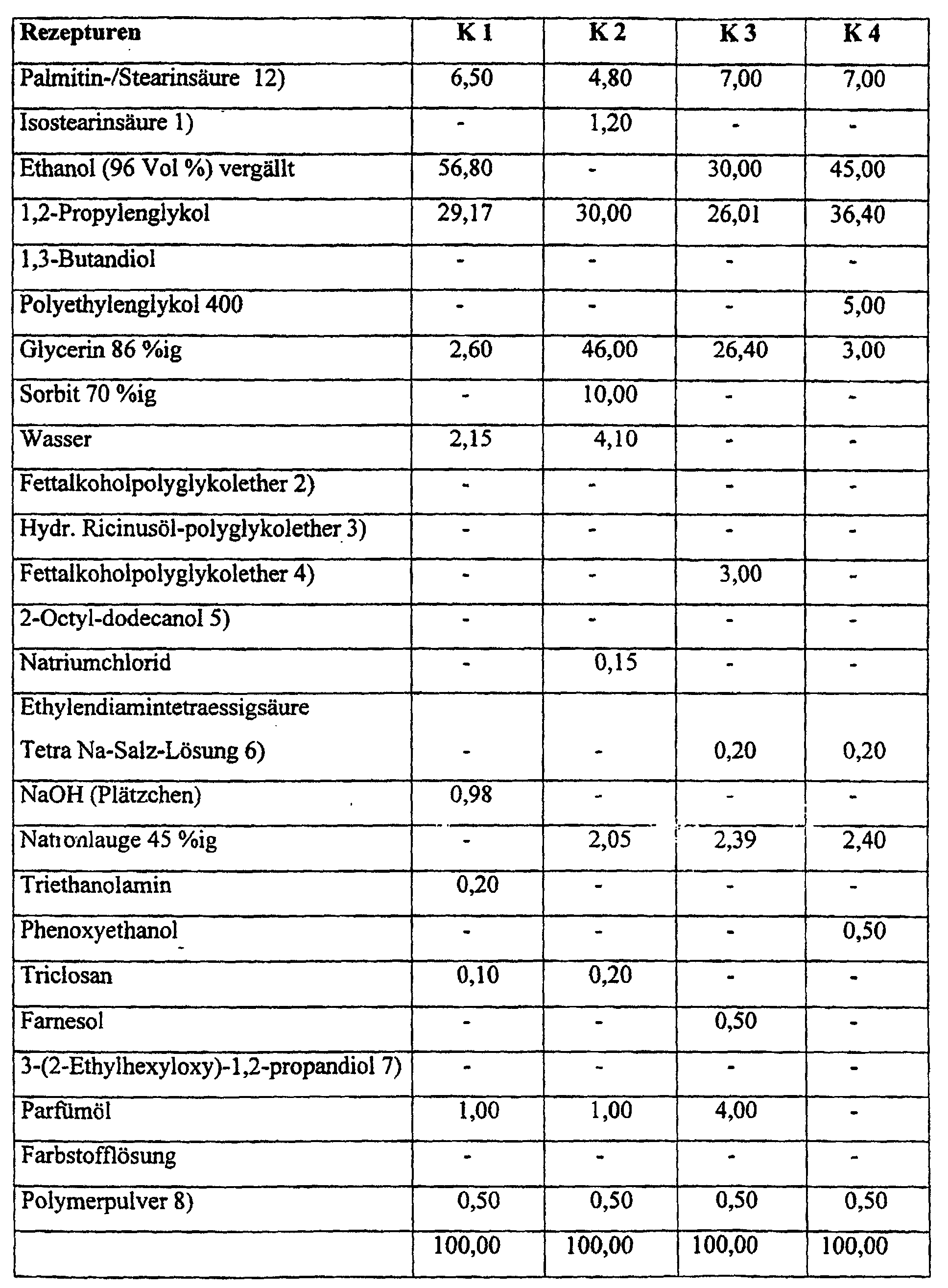

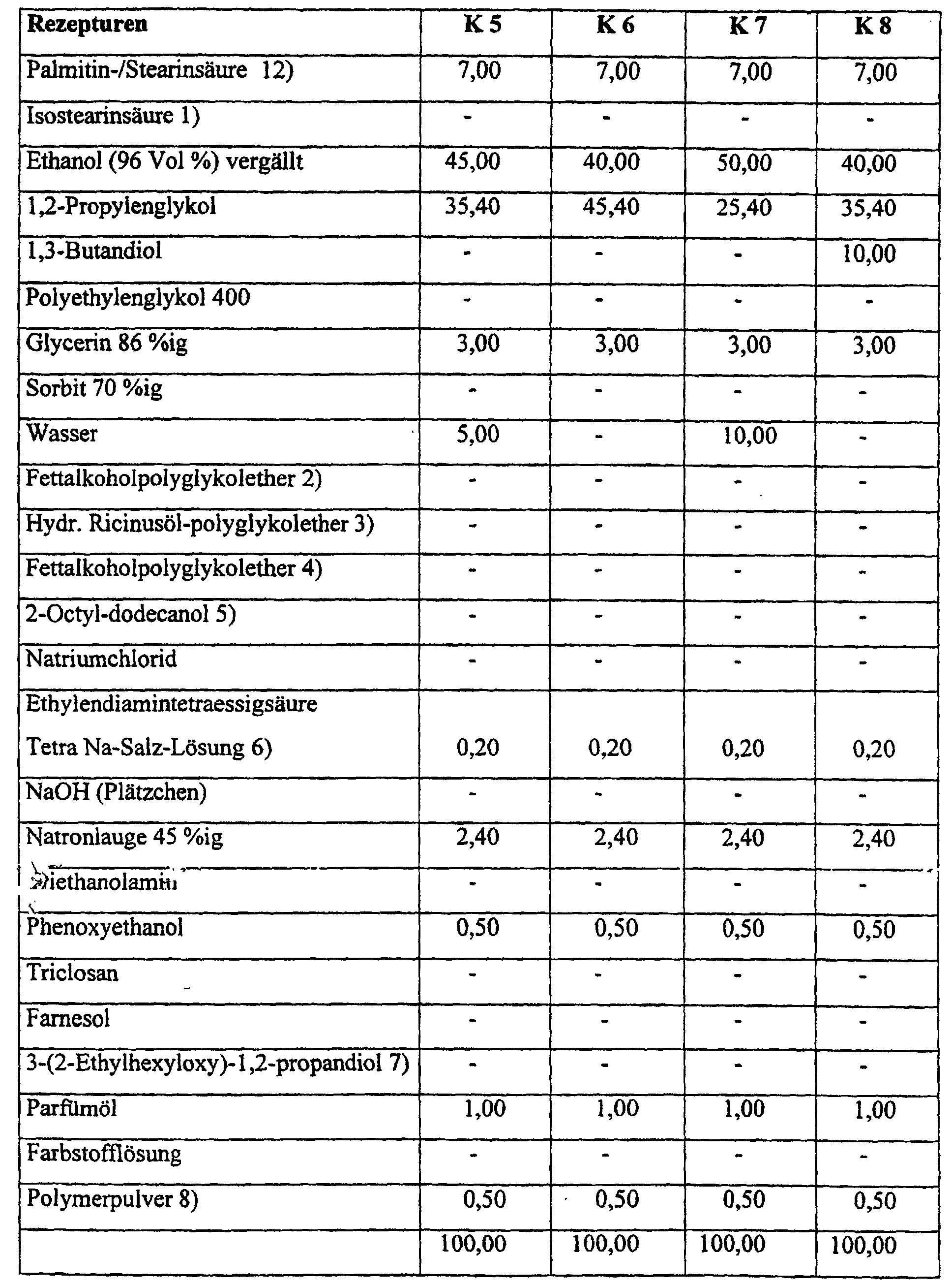

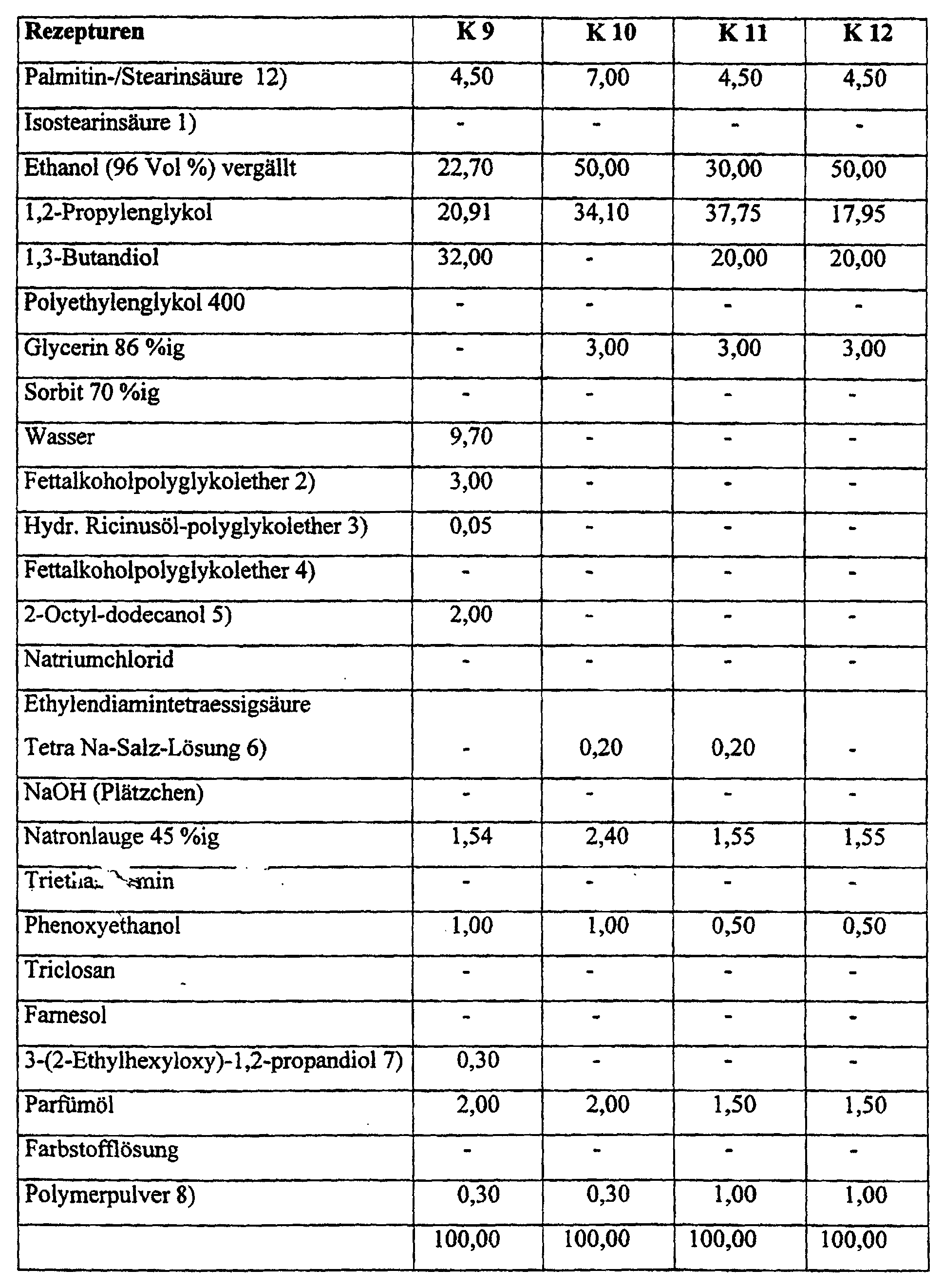

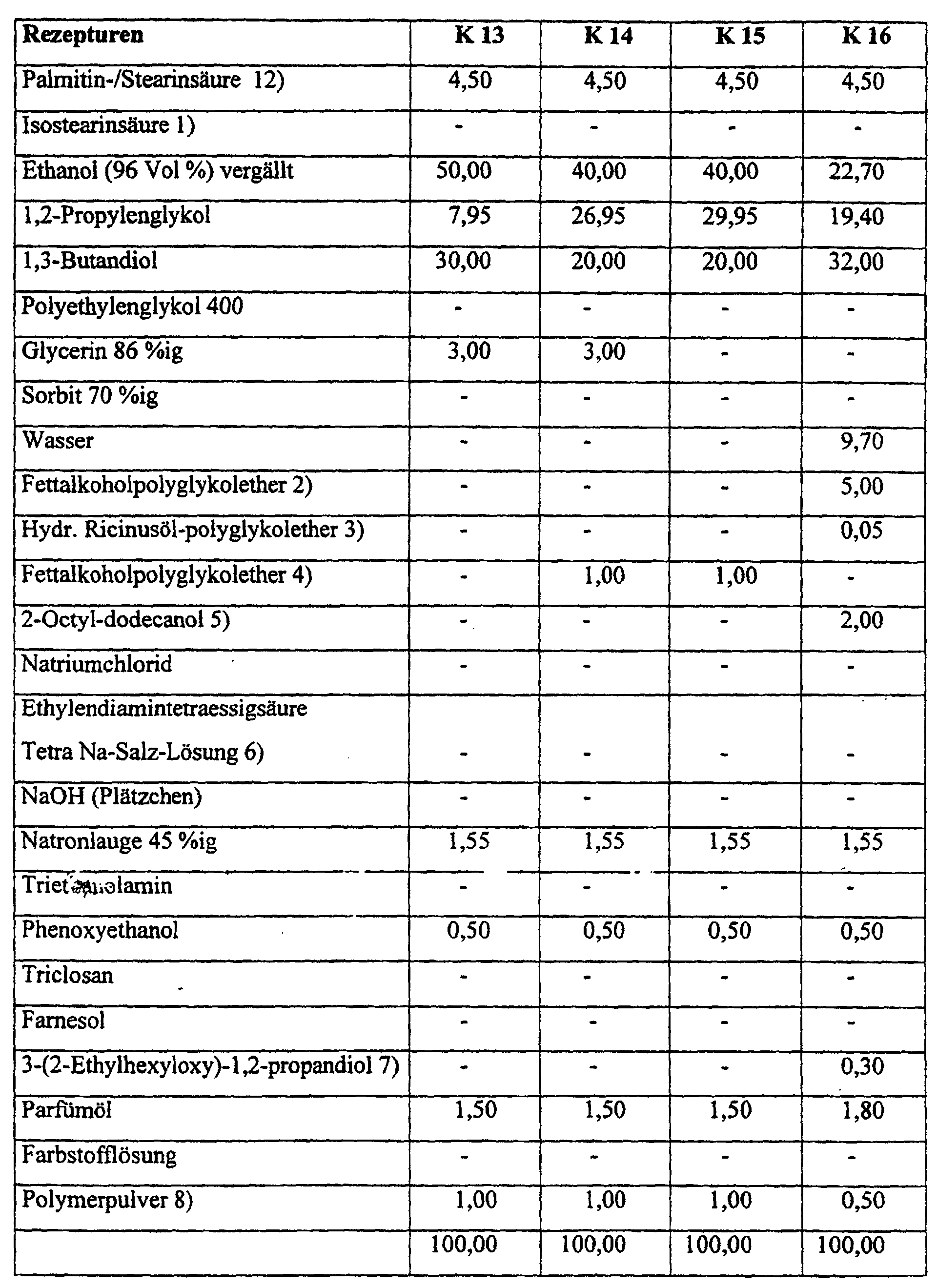

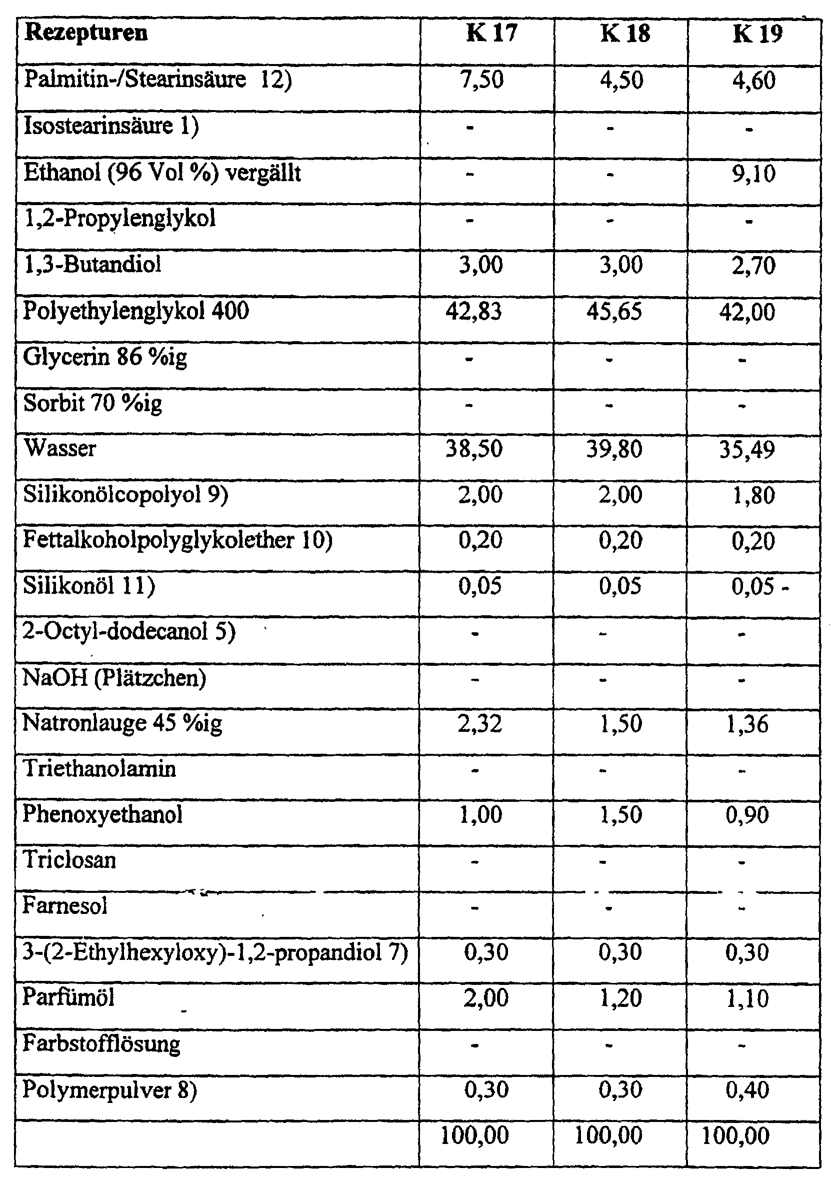

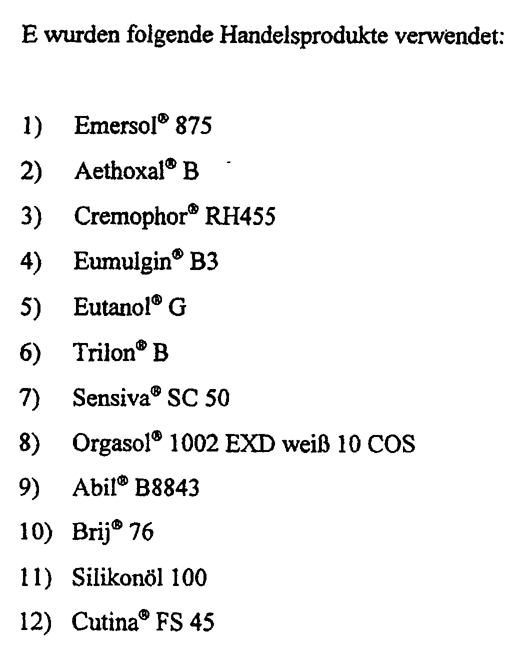

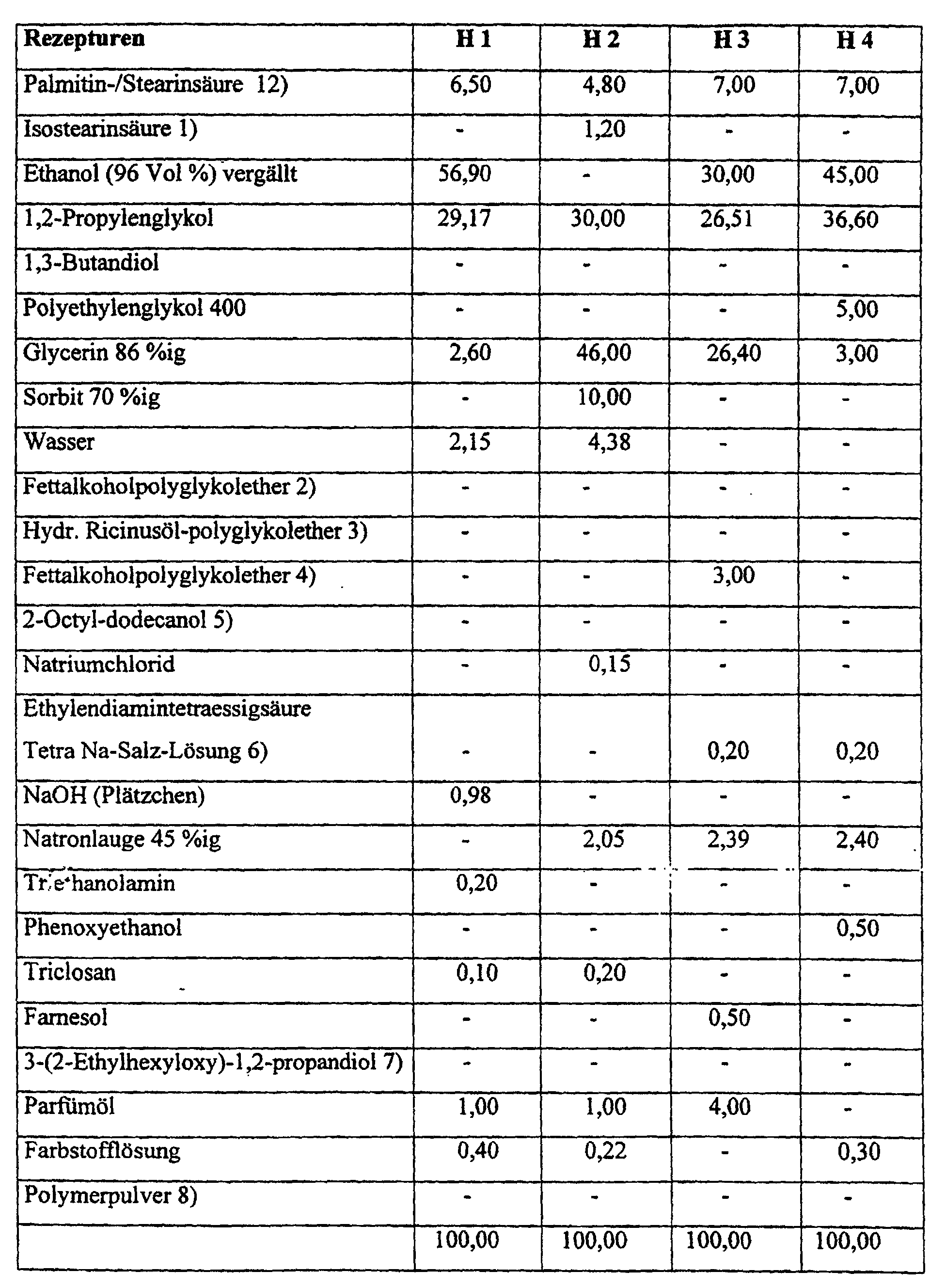

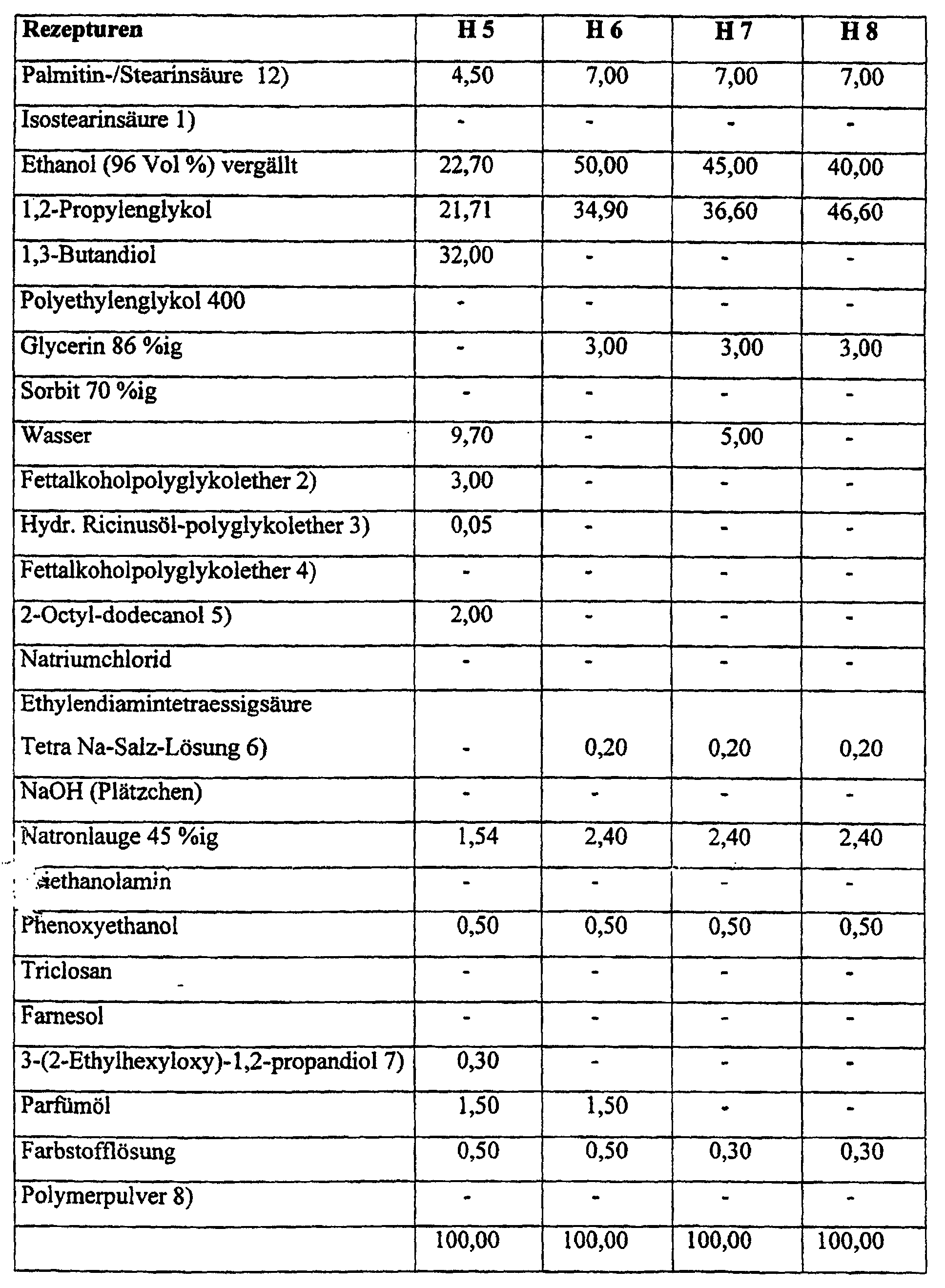

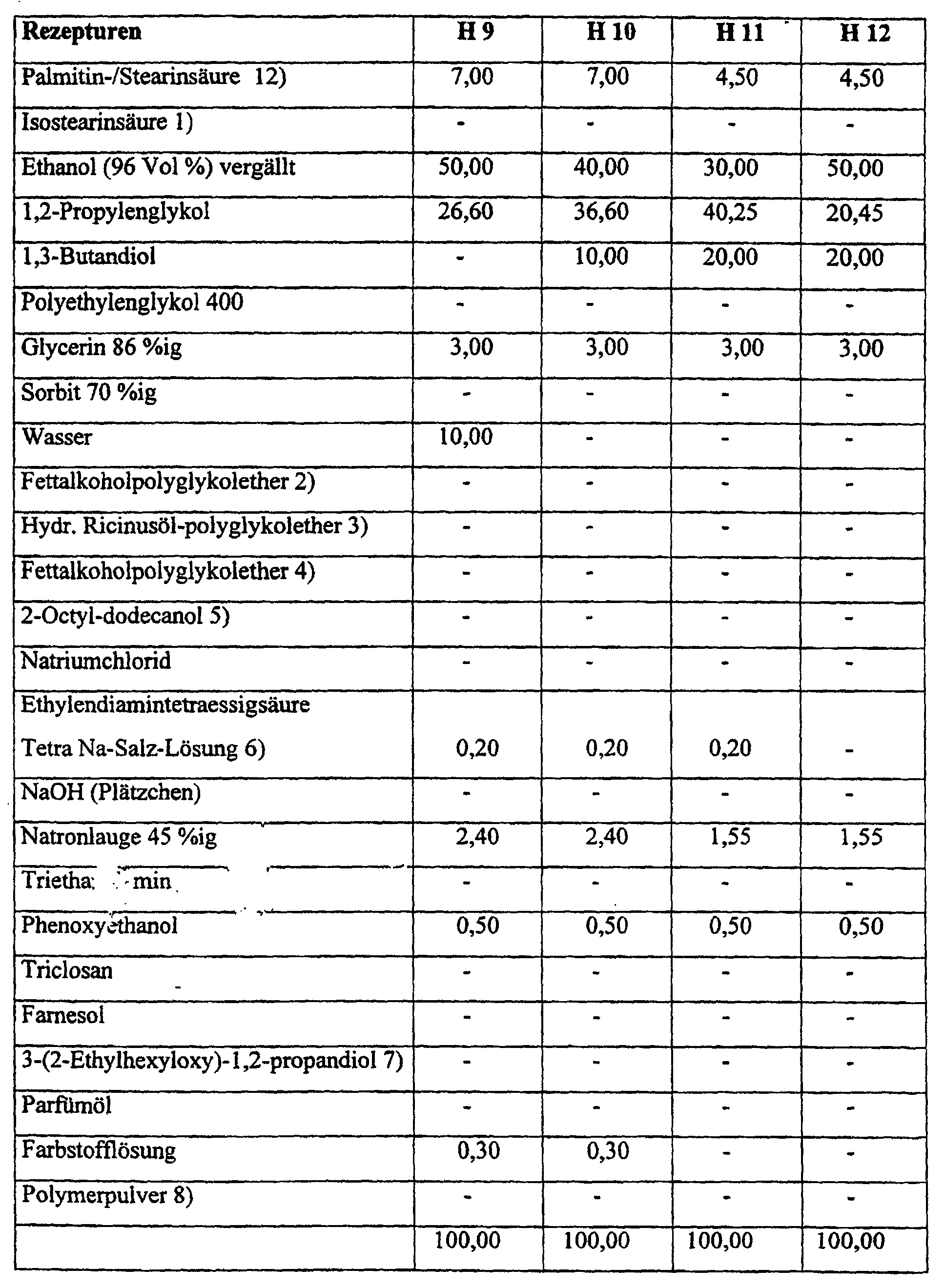

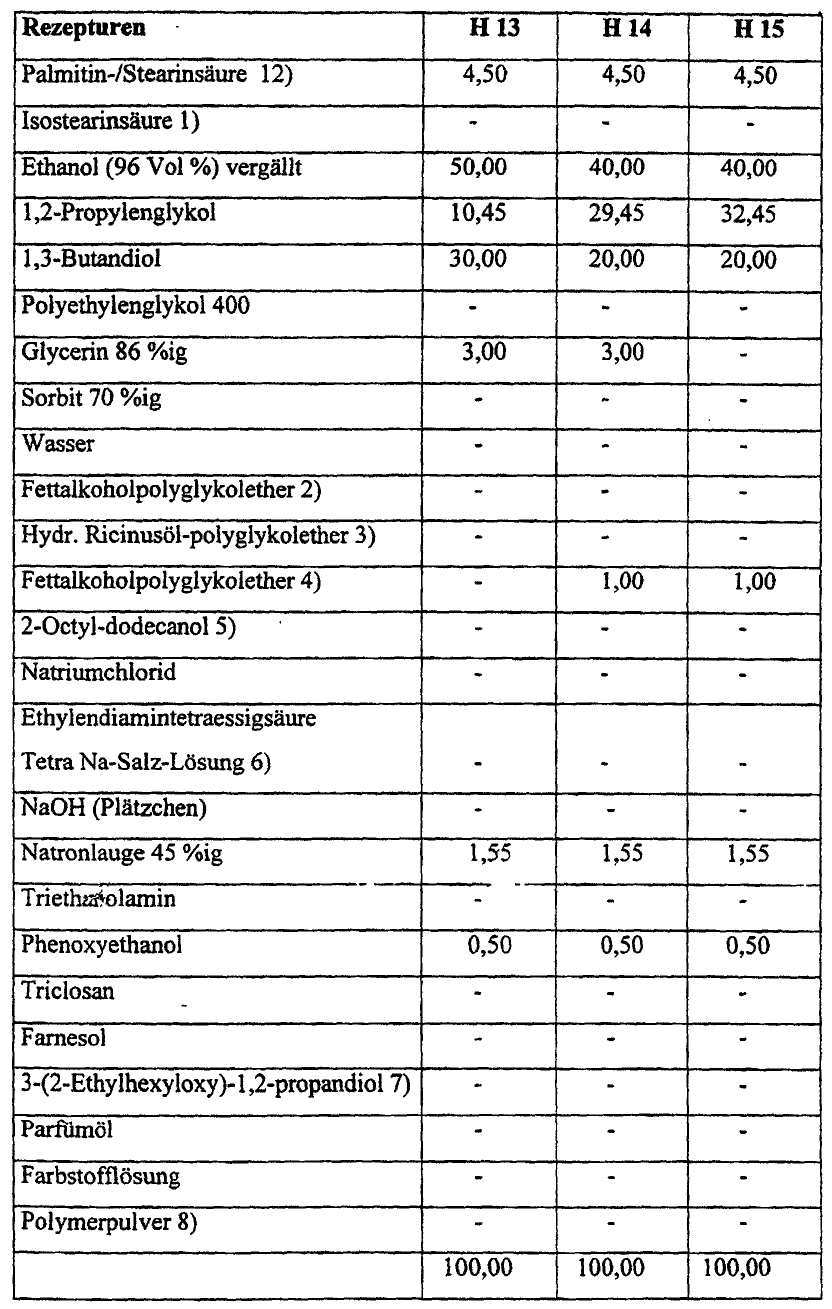

### 1. Herstellung der Stiftmassen mit Polymerpulver K 1 bis K 19

Zuerst wurde ein Vorgemisch aus
0,3 Gewichtsteilen Orgasol®1002 EXD weiß 10 COS
0,7 Gewichtsteilen 1,2-Propylenglycol
1,0 Gewichtsteilen Phenoxyethanol
0,3 Gewichtsteilen 3-(2-Ethylhexyloxy)-1,2-propandiol und
1,0 Gewichtsteilen Parfümöl
hergestellt. Dieses wurde als weiße, Nießfähige Paste erhalten.

Fettsäure, Ethanol und die restlichen Polyole und Ölkomponenten wurden gemischt, auf 65°C erwärmt und mit der auf 65°C erwärmten, 45 %igen wäßrigen Natronlauge versetzt. Die Verseifung wurde unter Rühren bei 65°C durchgeführt. Nach vollständiger Verseifung der Fettsäure wurde das Vorgemisch, das restliche Parfümöl und die übrigen Komponenten eingerührt. Danach wurde die Masse in vorbereitete zylindrische Formen abgefüllt, deren Durchmeser ca. 60% des Durchmessers einer handelsüblichen Kosmetik-Stiftmasse betrug.

Nach Abkühlung auf 15°C wurde die Masse entformt.

### 2. Herstellung der Stiftmassen H 1 bis H 15

Die Herstellung erfolgte in der für Seifengel-Stifte üblichen Weise.

### 3. Herstellung eines 2-Phasen-Deodorant-Stiftes

Eine entformte Stiftmasse gemäß Rezeptur K 4 wurde als Kern in eine übliche Stifthülse zentriert eingesetzt. Dann wurde die Stiftmasse H 4 hergestellt und bei 65°C in den Zwischenraum zwischen Kem und Hülsenwand eingegossen.

Nach Aufschrauben der Verschlußkappen wurden die Stifthülsen umgedreht, d.h. auf den Kopf gestellt, damit die noch nicht erstarrte Stiftmasse in der Verschlußkappe eine glatte Oberfläche ausbildet.

Nach Abkühlung wurde ein attraktiv aussehender 2-Phasen-Stift mit transparenter, leicht gefärbten äußerer Phase und weißem Kern erhalten.

In gleicher Weise wurden 2-Phasen-Stifte aus den folgenden Stiftmassen hergestellt:

| | |
|---|---|
| K 6 (Kern) + H 4 (Hülle) | K 8 (Kern) + H 10 (Hülle) |
| K 8 (Kern) + H 4 (Hülle) | K 5 (Kern) + H 4 (Hülle) |
| K 10 (Kern) + H 4 (Hülle) | K 7 (Kern) + H 15 (Hülle) |
| K 15 (Kern) + H 5 (Hülle) | K 9 (Kem) + H 5 (Hülle) |
| K 4 (Kern) + H 6 (Hülle) | K 11 (Kern) + H 5 (Hülle) |
| K 6 (Kern) + H 6 (Hülle) | K 18 (Kern) + H 5 (Hülle) |
| K 9 (Kern) + H 6 (Hülle) | K 5 (Kern) + H 6 (Hülle) |
| K 6 (Kern) + H 8 (Hülle) | K 8 (Kern) + H 6 (Hülle) |
| K 4 (Kern) + H 10 (Hülle) | K 10 (Kern) + H 6 (Hülle) |
| K 8 (Kern) + H 8 (Hülle) | K 8 (Kern) + H 8 (Hülle) |
| K 5 (Kern) + H 10 (Hülle) | K 10 (Kern) + H 10 (Hülle) |

In allen Fällen wurden 2-Phasen-Stifte mit deutlich erkennbarem Kern in einer transparenten Hülle erhalten, die über mehrere Wochen bei 25°C keine Veränderung zeigten.

## Patentansprüche

1. Stiftpräparat aus einer bis 40° C formstabilen, auf der Haut verstreichbaren und bei Temperaturen oberhalb 40° C schmelzbaren Masse, die aus zwei oder mehreren getrennten unterschiedlich zusammengesetzten Gelphasen besteht, die ein- oder mehrwertige Alkohole, Gelierungsmittel, Duftstoffe, kosmetische oder dermatologische Wirkstoffe sowie gegebenenfalls Wasser und galenische Hilfsmittel enthalten, **dadurch gekennzeichnet, daß** in einer der Gelphasen 0,1 - 10 Gew.-%, bezogen auf diese Phase, eines porösen Pulvers aus sphärischen Polymerteilchen dispergiert ist.

2. Stiftpräparat nach Anspruch 1, **dadurch gekennzeichnet, daß** die Masse in Form von zwei oder mehr konzentrisch angeordneten Phasen vorliegt, von welchen die innere oder eine der inneren Phasen das dispergierte Pulver aus sphärischen Polymerteilchen enthält.

3. Stiftpräparat nach einem der Ansprüche 1 oder 2, **dadurch gekennzeichnet, daß** die Phase
| | |
|---|---|
| 20 - 90 Gew.-% | einwertige oder ehrwertige Alkohole mit 2- 6 C-Atesen |
| 4 - 14 Gew.-% | Fettsäuren mit 12 - 22 C-Atomen in Form ihrer Metall- oder Aminseifen |
| 0,1 - 30 Gew.-% | Duftstoffe oder kosmetische oder dermatologische Wirkstoffe |
sowie gegebenenfalls Wasser und übliche galenische Hilfsmittel enthalten.

4. Stiftpräparat nach einem der Ansprüche 1 - 3, **dadurch gekennzeichnet, daß** die sphärischen Polymerteilchen einen Kern aus einem Pigment, bevorzugt aus Titandioxid, in einer Menge von 30 - 60 Gew.-%, bezogen auf die Teilchenmasse, besitzen.

5. Stiftpräparat nach einem der Ansprüche 1 - 4, **dadurch gekennzeichnet, daß** als kosmetische Wirkstoffe deodorierende oder transpirationshemmende Stoffe enthalten sind.

6. Verfahren zur Herstellung eines Stiftpräparats gemäß einem der Ansprüche 1 - 5, **dadurch gekennzeichnet, daß** man das poröse Pulver aus sphärischen Polymerteilchen und den Duftstoffen oder Wirkstoffen belädt, indem man sie in Form einer Dispersion in dem ein- oder mehrwertigen Alkohol, in der auch die Duftstoffe oder Wirkstoffe enthalten sind, in die Gelphase einarbeitet.

## Claims

1. A stick preparation of a composition which is dimensionally stable up to 40°C, can be spread onto the skin and melts at temperatures above 40°C and which consists of two or more separate gel phases of different composition which contain monohydric or polyhydric alcohols, gelling agents, perfumes, cosmetic or dermatological principles and optionally water and galenic auxiliaries, **characterized in that** a porous powder of spherical polymer particles is dispersed in one of the gel phases in a quantity of 0.1 to 10% by weight, based on that phase.

2. A stick preparation as claimed in claim 1, **characterized in that** it is present in the form of two or more concentric phases of which the inner phase or one of the inner phases contain(s) the dispersed powder of spherical polymer particles.

3. A stick preparation as claimed in claim 1 or 2, **characterized in that** the phases contain
| | |
|---|---|
| 20 to 90% by weight | of monohydric or polyhydric alcohols containing 2 to 6 carbon atoms, |
| 4 to 14% by weight | of fatty acids containing 12 to 22 carbon atoms in the form of their metal or amine soaps, |
| 0.1 to 30% by weight | of perfumes or cosmetic or dermatological principles |
and optionally water and typical galenic auxiliaries.

4. A stick preparation as claimed in any of claims 1 to 3, **characterized in that** the spherical polymer particles comprise a core of a pigment, preferably titanium dioxide, in a quantity of 30 to 60% by weight, based on the particle weight.

5. A stick preparation as claimed in any of claims 1 to 4, **characterized in that** deodorizing or perspiration-inhibiting agents are present as the cosmetic principles.

6. A process for producing the stick preparation claimed in any of claims 1 to 5, **characterized in that** the porous powder of spherical polymer particles is charged with the perfumes or active principles by incorporation in the gel phase in the form of a dispersion in the monohydric or polyhydric alcohol in which the perfumes or active principles are also present.

## Revendications

1. Préparation sous forme de bâton, à base d'une matière de forme stable à 40°C, pouvant s'étaler sur la peau et fusible à des températures supérieures à 40°C, qui consistent en deux ou plus de deux phases de gel séparées, de composition différente, qui contiennent des alcools mono- ou polyhydroxylés, des agents gélifiants, des parfums, des substances actives cosmétiques ou dermatologiques ainsi qu'éventuellement de l'eau et des adjuvants galéniques, dans l'une des phases de gel étant dispersés 0,1 à 10 % en poids, par rapport à cette phase, d'une poudre poreuse constituée de particules sphériques de polymère.

2. Préparation sous forme de bâton selon la revendication 1,
**caractérisée en ce que**
la matière se trouve sous forme de deux ou plus de deux phases concentriques, dont la phase interne ou l'une des phases internes contient la poudre dispersée à base de particules sphériques de polymère.

3. Préparation sous forme de bâton selon la revendication 1 ou 2,
**caractérisée en ce**
**que** la phase contient
| | |
|---|---|
| 20-90 % en poids | d'alcools monohydroxylés ou polyhydroxylés ayant de 2 à 6 atomes de carbone |
| 4-14 % en poids | d'acides gras ayant de 12 à 22 atomes de carbone, sous forme de leurs sels métalliques ou d'amines |
| 0,1-30 % en poids | de parfums ou de substances actives cosmétiques ou dermatologiques. |
ainsi qu'éventuellement de l'eau et des adjuvants galéniques usuels.

4. Préparation sous forme de bâton selon l'une quelconque des revendications 1 à 3,
**caractérisée en ce que**
les particules sphériques de polymère comportent un noyau à base d'un pigment, de préférence à base de dioxyde de titane, en une quantité de 30-60 %, par rapport à la masse des particules.

5. Préparation sous forme de bâton selon l'une quelconque des revendications 1 à 4.
**caractérisée en ce qu'**
elle contient en tant que substances actives cosmétiques des substances déodorantes ou antisudorales.

6. Procédé pour la fabrication d'une préparation sous forme de bâton selon l'une quelconque des revendications 1 à 5.
**caractérisé en ce qu'**
on charge la poudre poreuse à base de particules sphériques de polymère et des parfums ou des substances actives, en l'incorporant dans la phase de gel, sous forme d'une dispersion dans l'alcool mono- ou polyhydroxylés, dans laquelle sont également contenus les parfums ou les substances actives.
